(19) Europäisches Patentamt
European Patent Office
Office européen des brevets

(11) **EP 4 546 257 A1**

(12) **EUROPEAN PATENT APPLICATION**
published in accordance with Art. 153(4) EPC

(43) Date of publication:
**30.04.2025 Bulletin 2025/18**

(21) Application number: **23826975.7**

(22) Date of filing: **06.06.2023**

(51) International Patent Classification (IPC):
*G06T 7/00* (2017.01)　　*G06V 10/778* (2022.01)

(52) Cooperative Patent Classification (CPC):
**G06T 7/00; G06V 10/778**

(86) International application number:
**PCT/JP2023/021065**

(87) International publication number:
**WO 2023/248788 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.06.2022 JP 2022099357**

(71) Applicant: **HITACHI HIGH-TECH CORPORATION**
**Tokyo 105-6409 (JP)**

(72) Inventors:
• **HATTORI Hideharu**
**Tokyo 100-8280 (JP)**
• **KAKISHITA Yasuki**
**Tokyo 100-8280 (JP)**
• **SAKURAI Toshinari**
**Tokyo 105-6409 (JP)**
• **MAESHIMA Muneo**
**Tokyo 105-6409 (JP)**
• **OBARA Takayuki**
**Tokyo 105-6409 (JP)**

(74) Representative: **MERH-IP Matias Erny Reichl Hoffmann**
**Patentanwälte PartG mbB**
**Paul-Heyse-Straße 29**
**80336 München (DE)**

(54) **CLASSIFIER GENERATION DEVICE AND IMAGE DIAGNOSIS ASSISTANCE DEVICE**

(57)　An identifier creating device performs update of an identifier, identification basis analyzation, and analysis of a misidentification cause, updates the identifier using results of the analysis, and thus achieves creation of an identifier allowing an object in an image to be identified with high accuracy. The identifier creating device acquires an objective image, extracts, using an identifier, feature amount of the objective image from the objective image, identifies, using the identifier, the objective image from the feature amount and determines an identification value, determines an identification basis using the feature amount and the identification value, determines a portion to be improved using the identification basis and a teacher image, and updates the identifier based on the portion to be improved.

FIG. 1

EP 4 546 257 A1

**Description**

Incorporation by Reference

This application claims priority to Japanese Patent Application No. 2022-099357, filed on June 21, 2022, the contents of which are incorporated herein by reference.

Technical Field

The present invention relates to identifier creation and image diagnosis support, for example, an image processing technique using machine learning for detecting a specific object (e.g., cancer cell) contained in a captured image.

Background Art

Recently, image recognition techniques using machine learning etc. have been studied in the field of image recognition technology. Deep Learning etc. is used to improve detection accuracy of an object in an image. For example, Japanese Unexamined Patent Application Publication No. 2016-143351 proposes a technique for developing an identifier to detect an object in an image. In Japanese Unexamined Patent Application Publication No. 2016-143351, multiple groups of learning image data are set up for machine learning to calculate parameters of a neural network.

Summary of Invention

Technical Problem

However, even if learning images are divided into multiple image groups for re-learning to determine the parameters as in Japanese Unexamined Patent Application Publication No. 2016-143351, an image, which does not contribute to improving identification accuracy of the identifier, may be included in the image groups, and the identification accuracy of the identifier is thus not necessarily improved. Furthermore, in Japanese Unexamined Patent Application Publication No. 2016-143351, the cause of a reduction in identification accuracy of the identifier is unclear, and the accuracy of the identifier is thus not necessarily improved.

The invention, which has been made in view of such circumstances, provides a technique for creating an identifier that allows an object (e.g., abnormal tissue) in an image to be identified with high accuracy.

Solution to Problem

To solve the above problems, an identifier creating device according to one embodiment of the invention acquires an objective image, extracts, using an identifier, feature amount of the objective image from the objective image, identifies, using the identifier, the objective image based on the feature amount and determines an identification value, determines an identification basis using the feature amount and the identification value, determines a portion to be improved using the identification basis and a teacher image, and updates the identifier based on the portion to be improved.

Further features concerning the invention will be clarified from the content of this Description and the accompanying drawings. The embodiments of the invention are achieved by elements and combinations of various elements, the following detailed description, and aspects of the accompanying claims.

It should be understood that the content of the Description is merely exemplarily given, and is not intended to limit the claims or application examples of the invention in any sense.

Advantageous Effects of Invention

According to one embodiment of the invention, it is possible to create an identifier that allows an object (e.g., abnormal tissue) in an image to be identified with high accuracy. In addition, it is possible to present an identification basis of the identifier and quality (in numerical value) of the identification basis.

Brief Description of Drawings

Fig. 1 is a block diagram showing functions of an identifier creating device according to a first embodiment.

Fig. 2 shows an exemplary hardware configuration of the identifier creating device of the first embodiment or an image diagnosis support device according to a second embodiment.

Fig. 3 illustrates an exemplary operation of a feature extraction section.

Fig. 4 illustrates the exemplary operation of the feature extraction section.

Fig. 5 illustrates an exemplary operation of an identification section.

Fig. 6 illustrates the exemplary operation of the identification section.

Fig. 7 illustrates an exemplary operation of an identification basis analyzation section.

Fig. 8 illustrates an exemplary operation of an automatic cause analysis section.

Fig. 9A illustrates an exemplary display, by an output section, of an identification value (object-likelihood), identification/determination results, etc.

Fig. 9B illustrates an exemplary display, by the output section, of an identification value (identification score), identification basis, etc.

Fig. 10A illustrates an exemplary operation of the output section.

Fig. 10B shows an exemplary display of a determination result of performance of an identifier.

Fig. 11 is a flowchart for illustrating overall operation of the identifier creating device of the first embodiment.

Fig. 12 is a block diagram showing functions of an image diagnosis support device according to a second embodiment.

Fig. 13 is a flowchart illustrating overall operation of the image diagnosis support device of the second embodiment.

Fig. 14 is a schematic view of a configuration of a remote diagnosis support system equipped with the identifier creating device and the image diagnosis support device.

Fig. 15 is a schematic view of a configuration of an online contract service offering system equipped with the identifier creating device and the image diagnosis support device.

Description of Embodiments

According to one embodiment of the Description, there is provided an identifier creating device which, during machine learning, performs analyzation of an identification basis of an identifier while updating parameters of the identifier, automatically analyzes a cause of misidentification of the identifier using a teacher image and the identification basis, and updates the identifier through identification basis learning for reflecting results of the analysis on creation of the identifier, thereby achieves creation of an identifier allowing an object (e.g., abnormal tissue) in an image to be identified with high accuracy, and provided an identifier creating method that also achieves creation of such an identifier.

Even if a learning image database includes an image that does not contribute to improving identification accuracy of an identifier, a configuration is added so as to automatically analyze a cause of misidentification through identification processing of the identifier and analyzation of an identification basis, and the identifier is updated through identification basis learning for reflecting results of the analysis on creation of the identifier, making it possible to create an identifier allowing an object in an image to be identified with high accuracy. In addition, an identification basis of the identifier and quality (in numerical value) of the identification basis are presented.

Hereinafter, some embodiments of the Description will be described with reference to the accompanying drawings. In the drawings, functionally identical elements may be represented by the same numerals. Although the accompanying drawings show specific embodiments and implementation examples in accordance with the principles of the invention, these are merely intended for understanding of the invention and are in no way to be used to interpret the invention in a limiting manner.

While the embodiments have provided descriptions of the invention in sufficient detail for those skilled in the art to practice the invention, it should be understood that other implementations and modes are possible, and that the configuration and the structure can be modified, and various elements can be replaced without departing from the scope and spirit of the technical ideas of the invention. Therefore, the following description should not be construed as being limited to this.

Furthermore, the embodiments of the Description may be implemented as software running on a general-purpose computer, or as dedicated hardware, or a combination of software and hardware, as described later.

In the following, "each processing section (e.g., identification section) in a form of a program" is the subject (operating entity) to describe each processing in the embodiments of the Description, but since the program is executed by a processor (CPU, etc.) to perform prescribed processing while using a memory and communication ports (communication control device), the description may be performed with the processor as the subject.

(1) First Embodiment

Functional Configuration of Identifier Creating Device

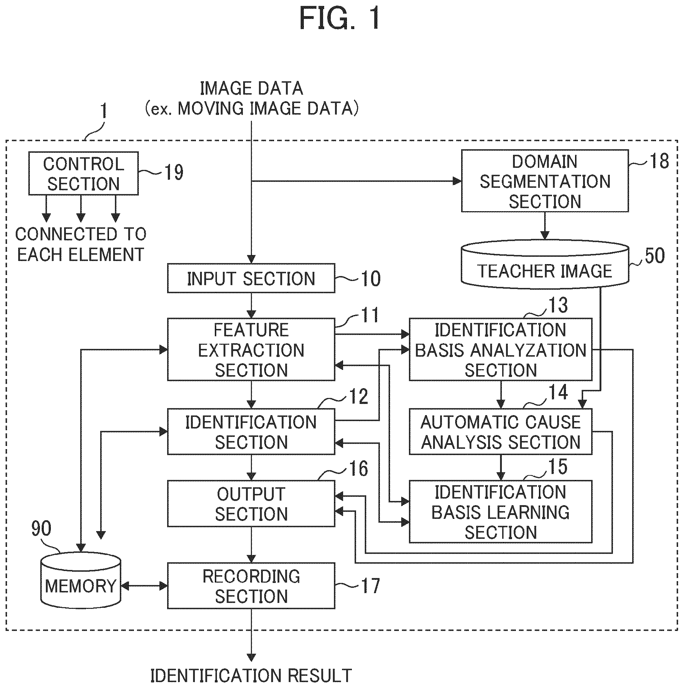

Fig. 1 is a block diagram showing a functional configuration of an identifier creating device according to a first embodiment of the Description. The identifier creating device 1 includes an input section 10, a feature extraction section 11, an identification section 12, an identification basis analyzation section 13, an automatic cause analysis section 14, an identification basis learning section 15, an output section 16, a recording section 17, a segmentation section 18, a control section 19, a teacher image (correct pattern, etc.) 50, and a memory 90.

If the teacher image beforehand exists in the identifier creating device 1, however, the segmentation section 18 is not a necessary component. The identifier creating device 1 may be implemented in an image diagnosis support device within an image acquisition device, or may be implemented within a server connected to the image acquisition device via a network, as described later (third and fourth embodiments).

In the identifier creating device 1, the identification section 12, the identification basis analyzation section 13, the automatic cause analysis section 14, the identification basis learning section 15, the output section 16, and the recording section 17 may be provided by a program or by a hardware module.

Image data is input into the input section 10. For example, the input section 10 may acquire still image data encoded in a format of JPG, Jpeg2000, PNG, BMP, or the like, Whole Slide Imaging (WSI), etc., and captured at a predetermined time interval by an imaging tool such as a camera built into the image acquisition device (not shown in Fig. 1), and may use that image as an input image.

The input section 10 may extract still image data of frames at a predetermined interval from video data in a format of Motion JPEG, MPEG, H.264, HD/SDI, or the like, and may acquire such an image as the input image. The input section 10 may use an image acquired by the imaging tool via a bus, a network, etc. as the input image. The input section 10 may acquire an image, which has been stored in a removable recording medium, as the input image. The input image and a teacher label are output from the input section 10 to the feature extraction section 11.

The feature extraction section 11 calculates feature amount (value of each parameter) from the input image and calculates an output value of each layer of a network (e.g., Convolutional Neural Network) configuring the feature extraction section 11.

The identification section 12 receives the output value of the final layer of the network of the feature extraction section 11, and calculates an identification result of the identifier.

The identification basis analyzation section 13 uses parameters of the feature extraction section 11 and the identification section 12 and the output value of each layer for the input image to calculate the identification basis (e.g., contribution rate (real number) of each feature amount to the identification result).

During machine learning to create the identifier, the automatic cause analysis section 14 uses the identification basis calculated by the identification basis analyzation section 13 and a teacher image 50 to analyze the cause of misidentification of the identifier, and thus calculates a mismatching portion (portion to be improved) between the identification basis and the teacher image, a portion to be improved in feature amount in a network of the identifier, and quality of the identification basis (matching degree between the identification basis and the teacher image).

The identification basis learning section 15 uses quality of the identification basis calculated by the automatic cause analysis section 14, parameters of the feature extraction section 11 and the identification section 12, an output value of each layer of the network of the identifier for an input image, and a teacher label (e.g., 0: normal tissue, 1: abnormal tissue) to perform machine learning so that an object in the input image is identified as an object, for example, a normal tissue or cell is identified as a normal tissue or cell, an abnormal tissue or cell in the input image is identified as an abnormal tissue or cell, and thus creates identifiers (parameters (filter coefficient, offset value, etc.) of the feature extraction section 11 and the identification section 12 required for identification) from the learning images.

The output section 16 displays information, such as an identification result calculated by the identification section 12, an identification basis calculated by the identification basis analyzation section 13, and quality of the identification basis calculated by the automatic cause analysis section 14, on an output unit (display, etc.).

The recording section 17 stores information, such as values calculated by the sections from the feature extraction section 11 to the identification basis learning section 15, in the memory 90.

The segmentation section 18 is not used when a teacher image exists in advance, but if no teacher image exists, it segments a domain in an objective image and creates a teacher image (correct pattern).

The control section 19 is implemented by a processor and is connected to each element within the identifier creating device 1. Operation of each element of the identifier creating device 1 is performed by autonomous operation of each of the above components or by an instruction from the control section 19.

As described above, in the identifier creating device 1 of this embodiment, during machine learning, while the feature extraction section 11 and the identification section 12 each update the parameter of the identifier using the learning image, the identification basis analyzation section 13 performs analyzation of the identification basis of the identifier using the parameters of the feature extraction section 11 and the identification section 12 and the output value of each layer for the input image. The automatic cause analysis section 14 automatically analyzes a cause of misidentification of the identifier using the teacher image and the identification basis, and the identification basis learning section 15 performs identification basis learning for reflecting results of the analysis on identifier creation and thus updates the identifier. In this way, the identifier creating device 1 is characterized by creating an identifier allowing an object (e.g., abnormal tissue) in an image to be identified with high accuracy.

Hardware Configuration of Identifier Creating Device

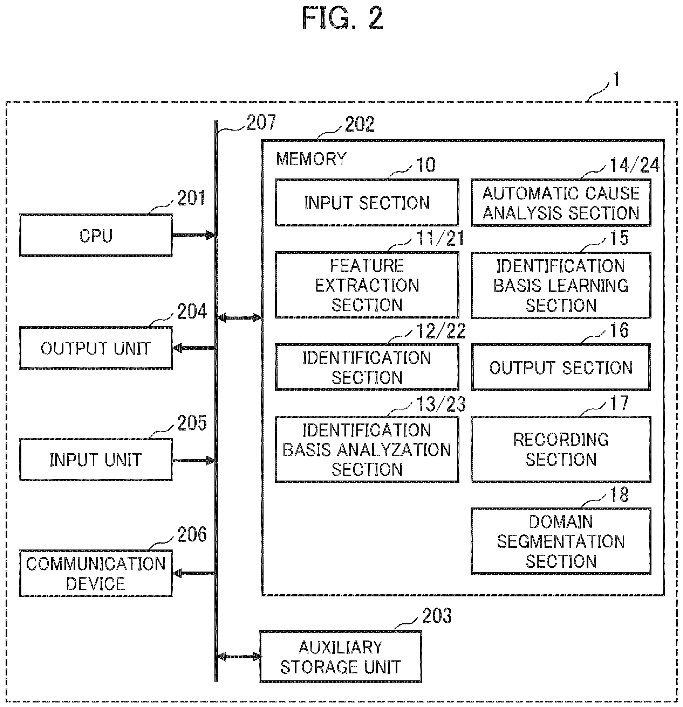

Fig. 2 shows an exemplary hardware configuration of the identifier creating device 1 according to this embodiment of the Description. The identifier creating device 1 includes CPU (processor) 201 that executes various programs, a memory (main storage unit) 202 that stores various programs, and an auxiliary storage unit 203 (corresponding to the memory 90) that stores various data. The identifier creating device 1 further includes an output unit 204 for outputting an identification result, an identification basis, quality of the identification basis, etc., an input unit 205 for inputting user instructions, images, etc., and a communication device 206 for communicating with other devices. These are interconnected by a bus 207.

The CPU 201 reads various programs from the memory 202 and executes the programs, as necessary. The memory 202 stores, in a form of programs, the input section 10, the feature extraction section 11, the identification section 12, the identification basis analyzation section 13, the automatic cause analysis section 14, the identification basis learning section 15, the output section 16, the recording section 17, and the segmentation section 18.

The auxiliary storage unit 203 stores: a learning image; an evaluation image; a teacher image; parameters , an identification result, and an identification value of the identifier created by the feature extraction section 11 and the identification section 12; identification basis (contribution rate (real number) of each feature amount to the identification result) created by the identification basis analyzation section 13; a mismatching portion (portion to be improved) between the identification basis and the teacher image, a portion to be improved in feature amount in the network of the identifier, and the quality of the identification basis (matching degree between the identification basis and the teacher image), which are created by the automatic cause analysis section 14; identifiers (parameters (filter coefficients, offset values, etc.) of the feature extraction section 11 and the identification section 12 necessary for identification) updated by the identification basis learning section 15; a teacher image, etc. created by the segmentation section 18; display information, etc. created by the output section 16.

The output unit 204 includes devices such as a display, a printer, and a speaker. For example, the output unit 204 displays the data created by the output section 16 on a display screen.

The input unit 205 includes devices such as a keyboard, a mouse, and a microphone. The input unit 205 is used to input user instructions (including determination of input of images including the teacher image) to the identifier creating device 1.

The communication device 206 is not an essential component for the identifier creating device 1, and if a personal computer or the like connected to the image acquisition device includes a communication device, the identifier creating device 1 may not include the communication device 206. For example, the communication device 206 receives data (including an image, the identifier, etc.) transmitted from another device (e.g., server) connected via a network, and stores the data in the auxiliary storage unit 203.

The identifier creating device of the Description performs analyzation of the identification basis of the identifier while updating parameters of the identifier, automatically analyzes a cause of misidentification of the identifier using the teacher image and the identification basis, and performs identification basis learning for reflecting results of the analysis on creation of the identifier and thus updates the identifier, thereby creates an identifier allowing an object (e.g., abnormal tissue) in an image to be identified with high accuracy.

Configuration and Operation of Each Section

The configuration and operation of each element will be described in detail below.

(i) Feature Extraction Section 11

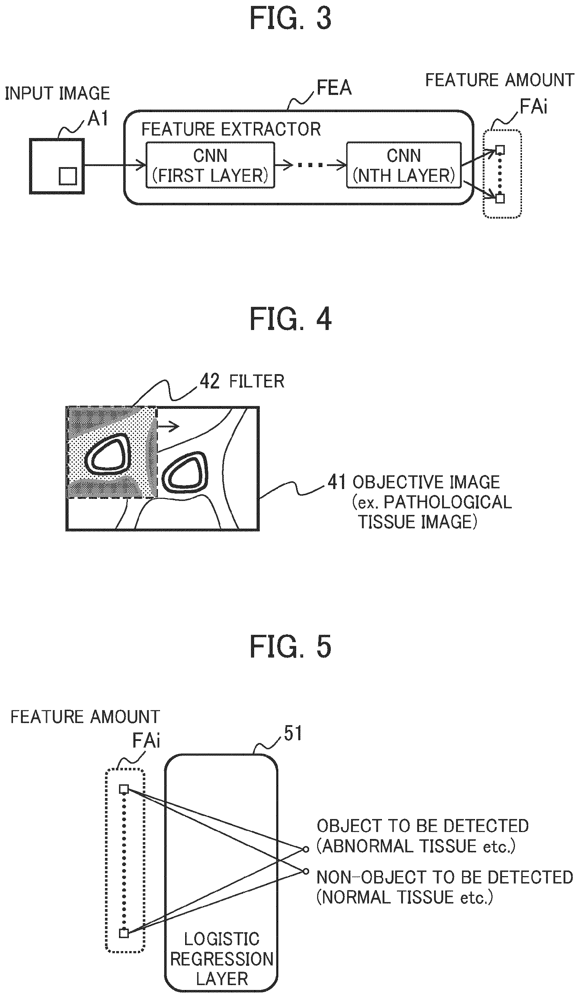

The feature extraction section 11 determines the feature amount of an input image. For example, Fig. 3 shows an exemplary determination of the feature amount. In Fig. 3, CNN represents Convolutional Neural Network. For example, the feature extraction section 11 uses a feature extractor FEA performing calculation of Formula (1) to determine feature amount FAi of an object (e.g., tissue or cell) in input image A1 from the input image A1.

The filter coefficient wj shown in Formula (1) is a coefficient determined through machine learning or the like so as to identify a non-object to be detected (e.g., identify a normal tissue or cell as a normal tissue or cell) as a non-object to be detected, and to identify the object to be detected as the object to be detected (e.g., identify an abnormal tissue or cell as an abnormal tissue or cell).

In Formula (1), pj represents a pixel value, bi represents offset value, m represents number of filter coefficients, and h represents nonlinear function. As shown in Fig. 4, the feature extraction section 11 uses Formula (1) to determine a calculation result of each filter 42 from the upper left to the lower right of an objective image 41, thereby determines the feature amount fi of any filter i. For example, a matrix of the feature amount fi determined by the feature extractor FEA is denoted as feature amount FAi of the input image A1. Explanation of the method for creating the feature extractor FEA is given in description of the identification section 12 later.
[Formula 1]

$$fi = h\left(\sum_{j=1}^{m} (pj \times wj) + bi\right) \quad \cdots \quad (1)$$

(ii) Identification Section 12

As shown in Fig. 5, the identification section 12 uses the feature amount FAi (matrix f) of the feature extractor FEA determined by the feature extraction section 11 to calculate a value of object-likelihood to be detected (abnormal tissue-likelihood, lesion-likelihood, etc.) according to Formula (2) by logistic regression process 51. The identification section 12 determines, based on the calculated value, whether an object (e.g., tissue or cell) in input image A1 is the object to be detected (abnormal tissue, etc.) or a non-object to be detected (normal tissue, etc.).

In Formula (2), w represents a weight matrix, b represents an offset value, g represents a nonlinear function, and y represents an identification result. As described later, the identification section 12 uses a learning image to determine the weight w and the offset value b through machine learning. In addition, the identification section 12 uses the weight w and the offset value b determined during learning to identify the objective image and calculate the identification result y.
[Formula 2]

$$y = g(w \times f + b) \quad \cdots \quad (2)$$

If the object in the input image is a non-object to be detected (e.g., a tissue or cell is a normal tissue or cell), the identification section 12, according to Formula (2), learns the feature amount of the object (e.g., tissue or cell) using the feature extraction section 11 with, for example, a known machine learning technique so that the object is determined to be a non-object to be detected (e.g., normal tissue or cell) by the logistic regression process 51, for example.

If the object in the input image is the object to be detected (e.g., a tissue or cell is an abnormal tissue or cell), the identification section 12 learns the feature amount of the object (e.g., tissue or cell) using the feature extraction section 11 so that the object is determined to be the object to be detected (e.g., abnormal tissue or cell) by the logistic regression process 51. For example, a Convolutional Neural Network may be used as the machine learning technique.

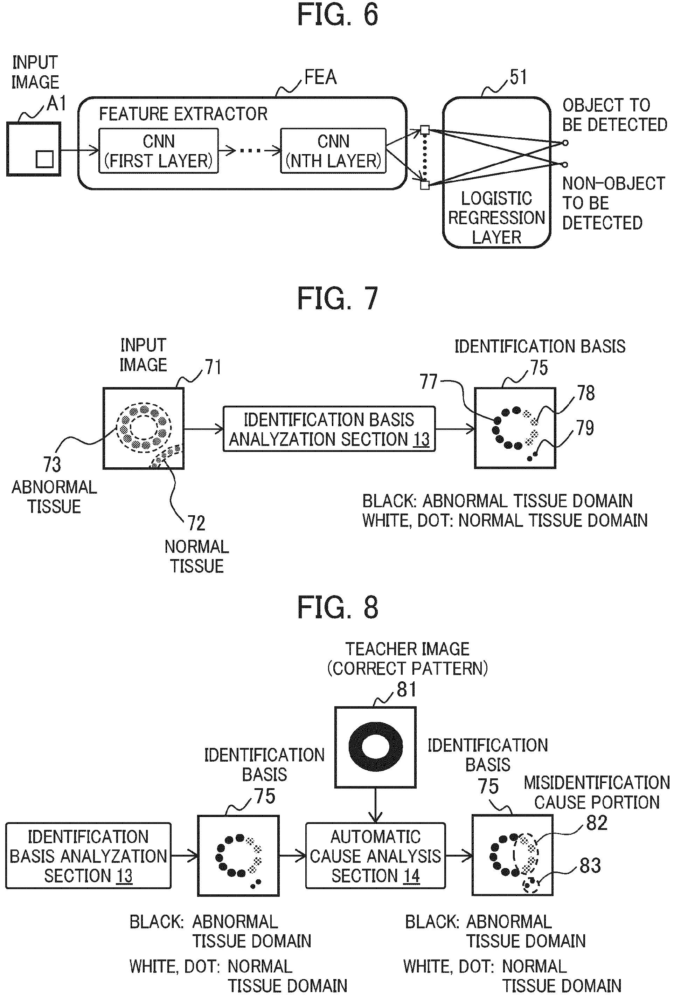

As shown in Fig. 6, during learning, the identification section 12, through machine learning, uses the input image A1 (e.g., HE-dyed image) to create the feature extractor FEA that calculates the feature amount fi (denoted as FAi) of the input image A1 using the feature extraction section 11 according to Formulas (1) and (2), so as to determine an object to be detected as the object to be detected (e.g., determine an abnormal tissue or cell as an abnormal tissue or cell), or determine a non-object to be detected is a non-object to be detected (e.g., determine a normal tissue or cell as a normal tissue or cell).

During learning, the identification section 12 repeatedly performs identification basis learning using multiple learning images with the feature extraction section 11, the identification section 12, and the identification basis learning section 15 described later, and thus determines the weight w, the filter coefficient wj, and the offset values b and bi as shown in Formulas (1) and (2), and creates the feature extractor FEA to calculate the feature amount FAi of the input image A1 from the input image A1, using the feature extraction section 11. Furthermore, the identification section 12 uses the feature extractor FEA to calculate an identification result of the objective image.

The identification section 12 stores the determined weights w, filter coefficient wj, and offset values b and bi in the memory 90.

(iii) Identification Basis Analyzation Section 13

Using a result z before applying the nonlinear function h in Formula (1), the identification basis analyzation section 13 calculates the identification basis (contribution rate (real number) of each feature amount with respect to the identification result) as shown in Fig. 7, according to Formulas (3) and (4).

In the example shown in Fig. 7, an input image 71 includes an abnormal tissue domain 73 and a normal tissue domain 72. Domains other than the domains 73 and 72 are also normal tissue domains. The identification basis 75 shows a domain of the identification basis of the abnormal tissue and a domain of the identification basis of the normal tissue. In the example of Fig. 7, the domain of the identification basis of the abnormal tissue is shown in black, and the domain of the identification basis of the normal tissue is shown in white or a dotted pattern. A domain 77 is a domain of the identification basis of the abnormal tissue included in the abnormal tissue domain 73. A domain 78 is a domain of the identification basis of the normal tissue included in the abnormal tissue domain 73. A domain 79 is a domain of the identification basis of the abnormal tissue included in the normal tissue domain 72.

In Formula (3), P represents input data from a previous layer consisting of pj, L is an index of a layer for which the identification basis is to be calculated, $C_L$ represents the identification basis of the Lth layer, and $C_{L+1}$ represents the identification basis of the (L+1)th layer. $W^{-1}$ represents a matrix obtained by replacing any dimension of the matrix of the filter coefficient wj in Formula (1) and inverting the direction of any dimension of the matrix. For example, $W^{-1}$ is a matrix obtained by replacing the first dimension (number of feature amounts) and the second dimension (number of channels) of the matrix of the filter coefficient wj in Formula (1) and inverting the direction of the third dimension (vertical size) and the direction of the fourth dimension (horizontal size).

[Formula 3]

$$C_L = \left(\frac{C_{L+1}}{z} \times W^{-1}\right) \times P \quad \cdots \quad (3)$$

The identification basis of a layer (e.g., Max Pooling layer) that acquires the maximum value among the layers of the identifier is that only a pixel where each domain ri has the maximum value is set to 1, and the rest are set to 0. Therefore, for example, the identification basis is propagated only to a pixel where the identification basis received from the adjacent layer on the output layer side is 1.

Assuming that the input data of the final layer of the identifier (e.g., Logistic Regression layer) is $P_{LR}$ and that label is a teacher signal in one hot vector format, the identification basis is calculated using Formula (4) and backpropagated to the previous layer of the identifier.

[Formula 4]

$$C_L = \left(\frac{label}{z} \times W^{-1}\right) \times P_{LR} \quad \cdots \quad (4)$$

(iv) Automatic Cause Analysis Section 14

The automatic cause analysis section 14 uses the identification basis calculated by the identification basis analyzation section 13 and a teacher image created manually or created by the segmentation section 18 to automatically analyze a domain causing misidentification as shown in Fig. 8. A teacher image (correct pattern) 81 is a monochrome image of the same size as the input image 71 and takes a value of 0 or 1, for example. For example, a domain of the teacher image with 0 represents a non-object domain (e.g., normal tissue domain), and a domain of that with 1 represents an object domain (e.g., abnormal tissue domain).

The non-object domain should be identified from feature amount within the non-object domain, and should not be identified with the object domain as the identification basis. The object domain (e.g., abnormal tissue domain) should be identified from feature amount within the object domain (e.g., abnormal tissue domain), and should not be identified with the non-object domain (e.g., normal tissue domain) as the identification basis.

Therefore, the automatic cause analysis section 14 compares the identification basis 75 calculated by the identification basis analyzation section 13 with the non-object domain and the object domain in the teacher image (correct pattern) 81, and specifies an identification basis, which appears in an inappropriate domain, as the cause of misidentification. Furthermore, the automatic cause analysis section 14 analyzes the cause of misidentification in different domains, i.e., the object domain and the non-object domain. In the example of Fig. 8, a portion 82 indicates a portion, where the identification basis of the abnormal tissue domain is lacking, in the abnormal tissue domain, and a portion 83 indicates a portion, where the identification basis of the abnormal tissue domain is shown, in the normal tissue domain.

The automatic cause analysis section 14 uses Formulas (5) and (6) to calculate a misidentification cause score $e_{back}$ for the non-object domain and a misidentification cause score $e_{fore}$ for the object domain. Here, $C_{sum}$ represents the identification basis for the object domain in the input image calculated by the identification basis analyzation section 13, and mask represents the teacher image. The identification basis $C_{sum}$ for the non-object domain may be used, and in such a case, a domain of the teacher image with 0 represents an object domain (e.g., abnormal tissue domain), and a domain thereof with 1 represents a non-object domain (e.g., normal tissue domain).

[Formula 5]

$$e_{back} = C_{sum} \times |(1 - mask) \quad \cdots \quad (5)$$

[Formula 6]

$$e_{fore} = -C_{sum} \left| \times mask \quad \cdots \quad (6) \right.$$

When the identification basis $C_{sum}$ exists in the non-object domain, feature amount, which is not suitable for identification, may be the identification basis, leading to a cause of a decrease in accuracy of the identifier. The automatic cause analysis section 14 therefore extracts the identification basis $C_{sum}$ included in the non-object domain, and calculates the score $e_{back}$ of the cause of misidentification in the non-object domain. The more the identification bases with positive values are included in the non-object domain, the larger the $e_{back}$ value.

The object domain contains feature amount for identifying the object domain, and desirably contains many identification bases $C_{sum}$ with positive values. Insufficient number of identification bases $C_{sum}$ in the object domain may cause an object to be missed. The automatic cause analysis section 14 therefore calculates the score $e_{fore}$ of the cause of misidentification of the object domain using the negative number of the identification basis $C_{sum}$ contained in the object domain.

The greater the proportion of the identification bases with negative values or small values, the greater the value of $e_{fore}$ will be, and the greater the proportion of the identification bases with positive values, the smaller the value of $e_{fore}$ will be. The automatic cause analysis section 14 may use, for example, Formulas (7) and (8) to calculate quality (matching degree between the identification basis and the teacher image) $coin_{back}$ of the identification basis outside the object domain and quality $coin_{fore}$ of the identification basis in the object domain. Here, X represents the number of horizontal pixels of the objective image, and Y represents the number of vertical pixels thereof.

[Formula 7]

$$coin_{back} = \frac{1}{XY} \sum_{x, y} (1 - Sigmoid(C_{sum})) \times (1 - mask) \quad \cdots \quad (7)$$

[Formula 8]

$$coin_{fore} = \frac{1}{XY} \sum_{x, y} Sigmoid(C_{sum}) \times mask \quad \cdots \quad (8)$$

A certain number of evaluation images are used to calculate $coin_{all}$ shown in Formula (9) for each identifier, and performance of the identifier is determined through comparison of $coin_{all}$ values. For example, it is determined that the larger the $coin_{all}$ value, the better the performance of the identifier.

[Formula 9]

$$coin_{all} = coin_{back} + coin_{fore} \quad \cdots \quad (9)$$

(v) Identification Basis Learning Section 15

The identification basis learning section 15 uses the score of the misidentification cause calculated by the automatic cause analysis section 14 and the identification result y calculated by the identification section 12 to perform identification basis learning for correcting the identification basis of the domain estimated as the misidentification cause. Multiple costs are used for the identification basis learning. For example, three types of costs can be used. The identification basis learning section 15 uses the following costs: first, Negative Log Likelihood (nll) of Formula (10) used in normal machine learning; second, cost $cgl_{back}$ on a cause of misidentification in the non-object domain as shown in Formula (11); and third, cost $cgl_{fore}$ on a cause of misidentification in the object domain as shown in Formula (12). In Formula (10), label represents a teacher label indicating the non-object domain or the object domain (e.g., 0 for the non-object domain and 1 for the object domain).

[Formula 10]

$$nll = -\{label \times \ln(y)\} \quad \cdots \quad (10)$$

[Formula 11]

$$cgl_{back} = \frac{1}{XY} \sum_{x,\,y} e_{back} \quad \cdots \quad (11)$$

[Formula 12]

$$cgl_{fore} = \frac{1}{XY} \sum_{x,\,y} e_{fore} \quad \cdots \quad (12)$$

The score $e_{back}$ of the misidentification cause calculated by the automatic cause analysis section 14 represents an identification basis contained in the non-object domain that should not be the identification basis. The identification basis learning section 15 therefore defines the average of $e_{back}$ calculated by the automatic cause analysis section 14 as the cost $cgl_{back}$ so that machine learning proceeds to reduce the value of $e_{back}$, and thus suppresses the identification basis in the non-object domain. The score $e_{fore}$ of the misidentification cause represents a domain having a low value of the identification basis in the object domain. The identification basis learning section 15 therefore increases the identification basis in the object domain by setting the average of $e_{fore}$, which is calculated by the automatic cause analysis section 14, as the cost $cgl_{fore}$.

The identification basis learning section 15 sets the sum of the three costs as the total cost cost as shown in Formula (13), performs machine learning to reduce cost, and while correcting the identification basis in the domain of the misidentification cause determined by the automatic cause analysis section 14, updates the parameters of the feature extraction section 11 and the identification section 12 to create the identifier allowing the object to be identified with high accuracy.

[Formula 13]

$$cost = \text{nll} + cgl_{back} + cgl_{fore} \quad \cdots \quad (13)$$

(vi) Output Section 16

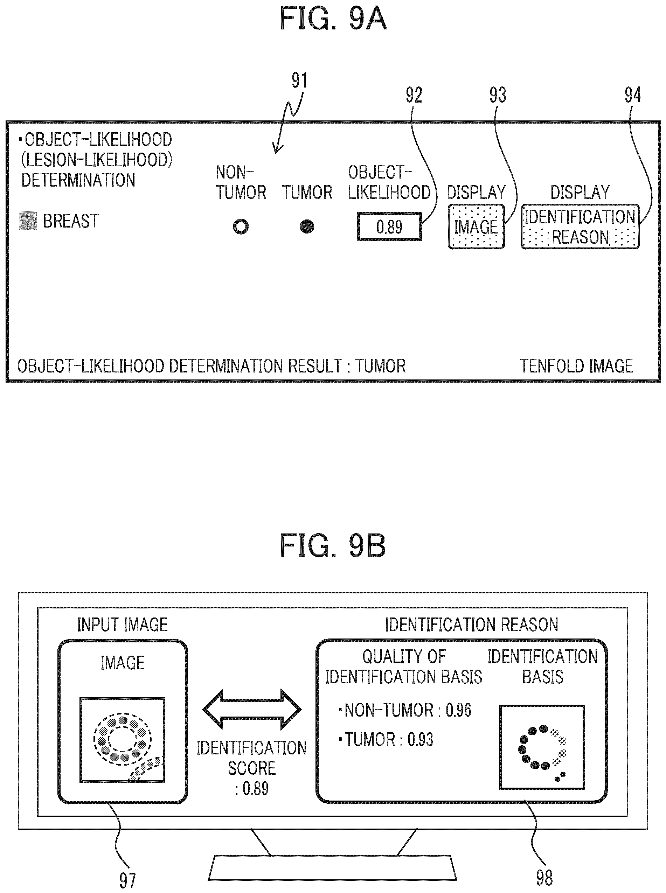

The output section 16 uses the identification result y determined by the created identifier to display, for example, a result of object-likelihood determination (e.g., lesion-likelihood determination), an identification basis, and quality of the identification basis in a graphical user interface (GUI) as shown in Figs. 9A and 9B.

The screen shown in Fig. 9A is displayed during use of the identifier, for example. Fig. 9A shows an exemplary case of a breast, showing a result of classifying whether non-tumor or tumor (abnormal tissue, abnormal cell, etc.). The exemplary screen of Fig. 9A displays a classification result 91, object-likelihood (tumor-likelihood) 92, an image display button 93, and an identification reason display button 94. When the image display button 93 is pressed by a user, for example, an input image is displayed. When the identification reason display button 94 is pressed, an identification reason is displayed.

In the exemplary case of Fig. 9A, the identification section 12, for the breast, classifies the input objective image to contain a tumor, which is an abnormal tissue or cell, and calculates an object-likelihood value of the tumor to be 0.89.

Fig. 9B is displayed during learning or use of the identifier, for example. Fig. 9B shows a display example of an input image, an identification basis for the input image, and the quality of the identification basis. Fig. 9B also shows an example in which quality of the identification basis of non-tumor is calculated to be 0.96, and quality of the identification basis of tumor is calculated to be 0.93. The screen of Fig. 9B may be displayed, for example, in response to operation of the image display button 93 or the identification reason display button 94 shown in Fig. 9A, or may be displayed independently of the screen of Fig. 9A.

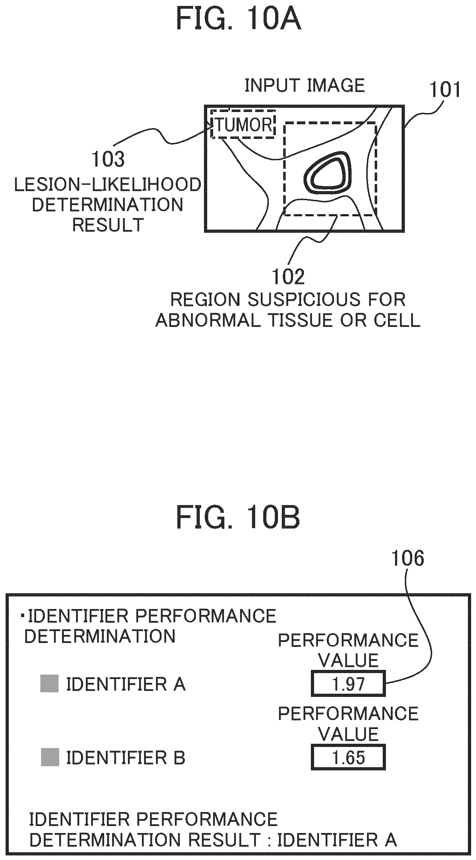

When receiving an unknown image from the input section 10 and displaying the identification result of each identifier, and if an object in the image is determined to be an object (e.g., abnormal tissue or cell), as shown in Fig. 10A, the output section 16 may draw a detection frame 102 within the received objective image 101 to indicate a portion of the object to be detected (e.g., portion suspicious for an abnormal tissue or cell). On the other hand, if the object is determined to be a normal tissue or cell, the output section 16 may display the received objective image 101 as it is without drawing the detection frame 102 on the received objective image 101. As shown in Fig. 10A, the output section 16 displays a result of determination on the object-likelihood (e.g., tumor).

The output section 16 uses a calculated performance value $coin_{all}$ of each identifier to determine performance of the identifier, and displays a determination result of performance of the identifier, for example, as shown in Fig. 10B. Fig. 10B is displayed during learning or use of the identifier, for example. Fig. 10B shows an example where the performance value of identifier A is displayed as 1.97, the performance value of identifier B is displayed as 1.65, and an identifier performance determination result is displayed as the identifier A.

The output section 16 is not an essential component for the identifier creating device 1, and if the image diagnosis support device includes an output section, the identifier creating device 1 may not have the output section 16.

(vii) Recording Section 17

The recording section 17 stores: in the memory 90, the input images (learning image, evaluation image, etc.); the teacher image; each parameter, the identification result, and the identification value of the identifier created by the feature extraction section 11 and the identification section 12; and the identification basis (contribution rate (real number) of each feature amount to the identification result) created by the identification basis analyzation section 13. The recording section 17 further stores, in the memory 90, a mismatching portion (portion to be improved) between the identification basis and the teacher image, a portion to be improved in feature amount in a network of the identifier, and quality of the identification basis (matching degree between the identification basis and the teacher image) created by the automatic cause analysis section 14. The recording section 17 also stores, in the memory 90, the identifiers (parameters (filter coefficients, weights, offset values, etc.) of the feature extraction section 11 and the identification section 12 necessary for identification) updated by the identification basis learning section 15, teacher image, etc. created by the segmentation section 18, and display information, etc. created by the output section 16.

The segmentation section 18 uses a segmentation method such as U-Net to segment each pixel in the input image into domains (e.g., object domain, non-object domain), and creates the teacher image.

Procedure of Identifier Creating Device

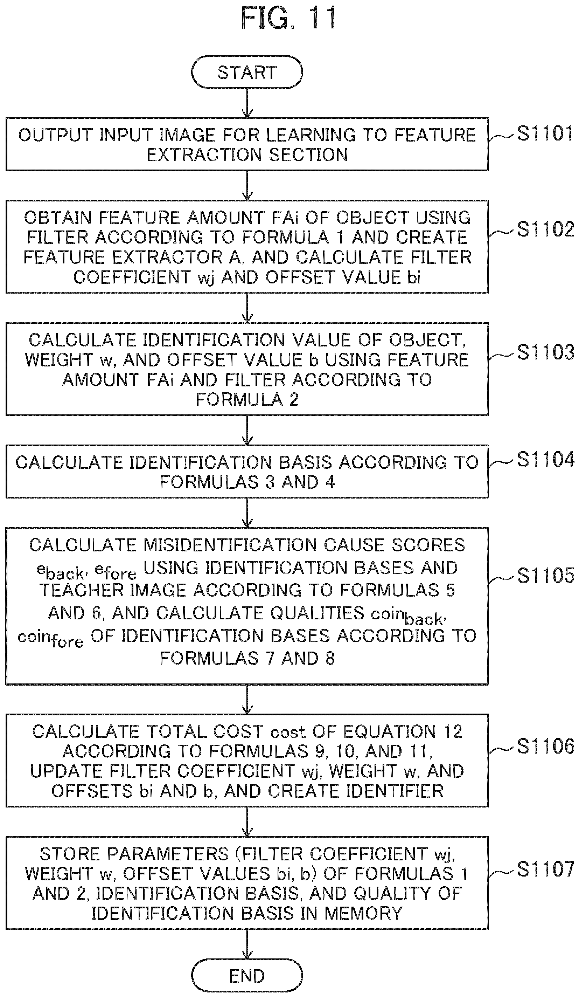

Fig. 11 is a flowchart for explaining operation of the identifier creating device 1 according to this embodiment of the Description. The following description is given with each processing section (input section 10, feature extraction section 11, etc.) as the operation subject, which however may be interpreted as follows: The CPU 201 is the operation subject and executes each processing section in a form of a program.

(i) Step 1101

The input section 10 receives a learning image and outputs the received image to the feature extraction section 11.

(ii) Step 1102

The feature extraction section 11, through machine learning, determines the feature amount FAi of an object (e.g., tissue or cell) in the input image A1 using a filter according to Formula 1, and creates the feature extractor FEA. The feature extraction section 11 determines the filter coefficient wj and the offset value bi for the feature amount FAi.

(iii) Step 1103

The identification section 12, through machine learning, determines an identification result from the feature amount FAi according to Formula 2, calculates an identification value for object-likelihood (e.g., lesion-likelihood), and determines each parameter (weight w, offset value b, etc.) of Formula 2 for determining the identification value so as to determine whether an object in the input image A1 is an object to be detected (e.g., normal cell or abnormal cell).

(iv) Step 1104

The identification basis analyzation section 13 determines the identification basis of an objective image according to Formulas 3 and 4.

(v) Step 1105

The automatic cause analysis section 14 uses the identification basis calculated by the identification basis analyzation section 13 and the teacher image to calculate misidentification cause scores, $e_{back}$ and $e_{fore}$, according to Formulas 5 and 6, and determines the qualities $coin_{back}$ and $coin_{fore}$ of the identification bases according to Formulas 7 and 8.

(vi) Step 1106

The identification basis learning section 15 calculates the total cost cost of Formula 12 according to Formulas 9, 10, and 11, updates the filter coefficient wj, the weight w, and the offsets bi and b using machine learning to reduce cost, and creates an identifier.

(vii) Step 1107

The recording section 17 stores, in the memory 90, respective parameters (filter coefficient wj, weight w, offset values bi and b, etc.) of Formulas 1 and 2, the identification basis, and the quality of the identification basis.

According to the first embodiment, during machine learning, analyzation of the identification basis of the identifier is performed while the parameters of the identifier are updated, a cause of misidentification of the identifier is automatically analyzed using the teacher image and the identification basis, and the identifier is updated through the identification basis learning for reflecting results of the analysis on creation of the identifier, making it possible to create an identifier that allows an object (e.g., abnormal tissue) in an image to be identified with high accuracy.

It is also possible to present the identification basis showing the reason for identifying the objective image and quality of the identification basis showing accuracy of the identifier.

(2) Second Embodiment

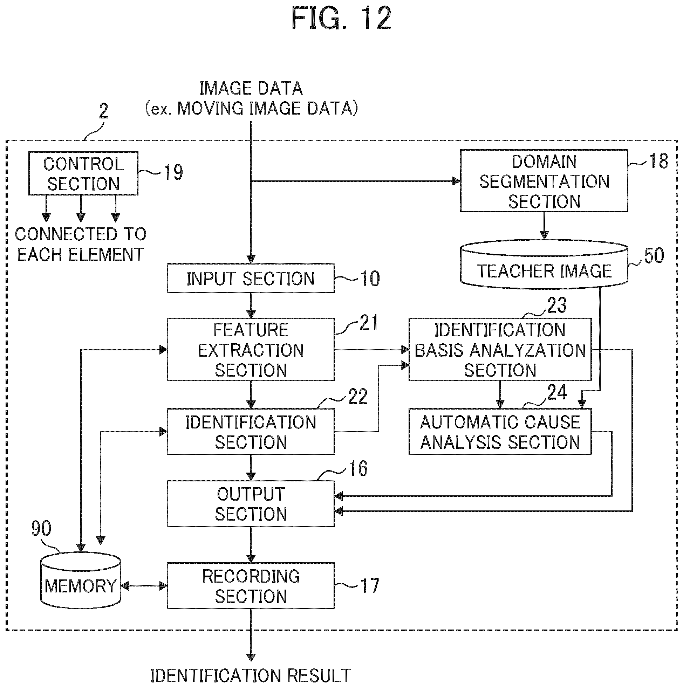

As shown in Fig. 12, an image diagnosis support device 2 according to a second embodiment largely includes the same components as the identifier creating device 1 of Fig. 1 of the first embodiment, however, unlike the first embodiment, includes no identification basis learning section 15, but includes the segmentation section 18, a feature extraction section 21, an identification section 22, an identification basis analyzation section 23, and an automatic cause analysis section 24. Therefore, only the components different from those in Fig. 1 are described herein.

The image diagnosis support device 2 of this embodiment of the Description reads each parameter of an identifier from the memory, and the segmentation section 18 creates a teacher image for an evaluation image by segmentation. Furthermore, the identification basis analyzation section 23 performs analyzation of an identification basis of the evaluation image. The automatic cause analysis section 24 automatically analyzes a cause of misidentification of the identifier using the teacher image and the identification basis, and performs analyzation of quality of the identification basis of the evaluation image, and thus presents an identification result of an object **(e.g.,** tissue or cell) in the evaluation image, the identification basis, and the quality of the identification basis.

Configuration and Operation of Each Section

The configuration and operation of each element that differs from that in Fig. 1 are described in detail below.

(i) Segmentation Section 18

The segmentation section 18 uses a segmentation model (e.g., U-Net), which segments an object domain, a non-object domain, and the like in an evaluation image, to perform segmentation of the evaluation image, and thus creates a teacher image.

(ii) Feature Extraction Section 21

The feature extraction section 21 reads each parameter of the learned feature extractor from the memory 90, and uses the feature extractor to calculate the feature amount FAi of the evaluation image according to Formula 1.

(iii) Identification Section 22

The identification section 22 reads each parameter of the learned identifier from the memory 90, and uses the identifier to identify the evaluation image from the feature amount FAi according to Formula 2, and calculates the identification result y and the identification value.

(iv) Identification Basis Analyzation Section 23

The identification basis analyzation section 23 determines an identification basis of the evaluation image according to Formulas 3 and 4.

(v) Automatic Cause Analysis Section 24

The automatic cause analysis section 24, according to Formulas 5 and 6, uses the identification basis calculated by the identification basis analyzation section 23 and the teacher image (correct pattern), which is determined by the segmentation section 18, for the evaluation image to calculate a portion of mismatching between the identification basis and the teacher image (portion to be improved), a misidentification cause score, and quality of the identification basis (matching degree between the identification basis and the teacher image).

Hardware Configuration of Image Diagnosis Support Device

An exemplary hardware configuration of the image diagnosis support device 2 according to this embodiment of the Description has a configuration similar to that of Fig. 2, but unlike the identifier creating device 1 of the first embodiment, the memory 202 stores the segmentation section 18, the feature extraction section 21, the identification section 22, the identification basis analyzation section 23, and the automatic cause analysis section 24.

The auxiliary storage unit 203 of the image diagnosis support device 2 stores an identification result y determined by the identification section 22, an identification basis determined by the identification basis analyzation section 23, and quality of the identification basis determined by the automatic cause analysis section 24, etc.

Procedure of Image Diagnosis Support Device

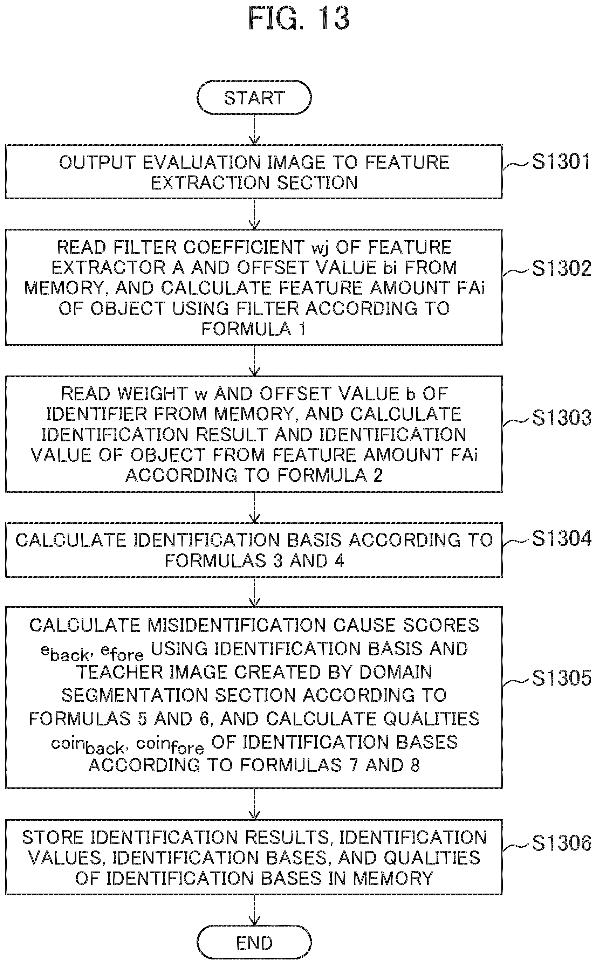

Fig. 13 is a flowchart for explaining operation of the image diagnosis support device 2 according to this embodiment of the Description. The following description is given with each processing section (input section 10, feature extraction section 21, etc.) as the operation subject, which however may be interpreted as follows: The CPU 201 is the operation subject and executes each processing section in a form of a program.

(i) Step 1301

The input section 10 receives the evaluation image and outputs the received image to the feature extraction section 21.

(ii) Step 1302

The feature extraction section 21 reads the filter coefficient wj and the offset value bi of the learned feature extractor FEA from the memory 90, and uses a filter to determine the feature amount FAi of an object (e.g., tissue or cell) in the input image A1 according to Formula 1.

(iii) Step 1303

The identification section 22 reads each parameter (weight w, offset value b, etc.) of the learned identifier from the memory 90, determines an identification result from the feature amount FAi according to Formula 2, calculates the identification value of object-likelihood (e.g., lesion-likelihood), and determines whether the object in the input image A1 is an object to be detected (e.g., normal cell or abnormal cell).

(iv) Step 1304

The identification basis analyzation section 23 determines the identification basis of the objective image according to Formulas 3 and 4.

(v) Step 1305

The automatic cause analysis section 24 uses the identification bases calculated by the identification basis analyzation section 23 and the teacher image for the objective image determined by the segmentation section 18 to calculate misidentification cause scores, $e_{back}$ and $e_{fore}$, according to Formulas 5 and 6, and determines the qualities $coin_{back}$ and $coin_{fore}$ of the identification bases according to Formulas 7 and 8.

(vi) Step 1306

The recording section 17 stores the identification result, the identification values, the identification bases, and the qualities of the identification bases in the memory 90.

According to the second embodiment, each parameter of the identifier is read from the memory, the teacher image for the evaluation image is created through segmentation, and analyzation of the quality of the identification basis of the evaluation image is performed based on the identifier using the teacher image and the identification basis while analyzation of the identification basis of the evaluation image is performed, making it possible to present the identification result of an object (e.g., tissue or cell) in the evaluation image, the identification basis, and the quality of the identification basis.

While the identifier is changed to another identifier in another facility, etc., the identification basis of the evaluation image and the quality of the identification basis are presented, making it possible to determine performance of an identifier created elsewhere. In addition, the identification basis of the evaluation image and the quality of the identification basis are presented while multiple identifiers are switched therebetween, making it possible to determine which identifier can be used to identify the object in the evaluation image with high accuracy.

(3) Third Embodiment

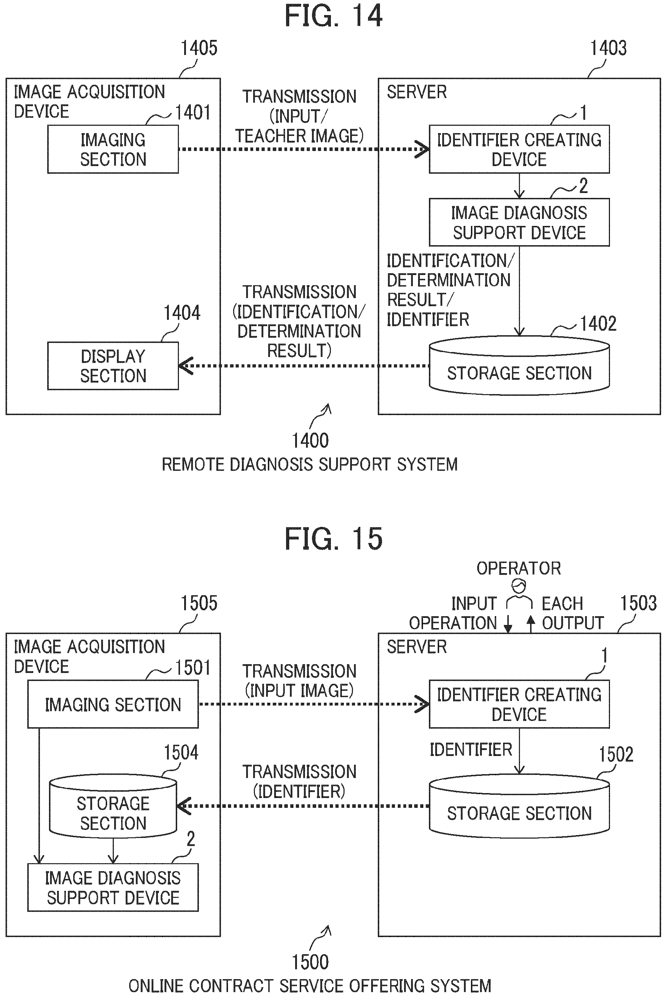

Fig. 14 is a functional block diagram showing a configuration of a remote diagnosis support system 1400 according to a third embodiment of the Description. The remote diagnosis support system 1400 includes a server 1403 and an image acquisition device 1405. The server 1403 can be configured of, for example, one or more computers.

The image acquisition device 1405, which is, for example, a virtual slide device or a personal computer equipped with a camera, includes an imaging section 1401 to capture image data and a display section 1404 to display identification/determination results transmitted from the server 1403. Although not shown, the image acquisition device 1405 includes a communication device that transmits image data to the server 1403 and receives data transmitted from the server 1403.

The server 1403 includes the image diagnosis support device 2 that creates the identifier using the identifier creating device 1 of the first embodiment of the Description for the image data (input image, teacher image) transmitted from the image acquisition device 1405, and performs image processing using the created identifier, and a storage section 1402 to store dentification/determination results and an identification basis/quality of the identification basis outputted from the image diagnosis support device 2. Although not shown, the server 1403 includes a communication device that receives image data transmitted from the image acquisition device 1405, and transmits data, such as the identification/determination results and the identification basis/quality of the identification basis, to the image acquisition device 1405.

The image diagnosis support device 2 uses the identifier obtained by the identifier creating device 1 to classify whether an object to be detected (e.g., abnormal tissue or cell (e.g., cancer)) exists in an object (e.g., tissue or cell) in the image data captured by the imaging section 1401. The display section 1404 displays the data of identification/evaluation results, an identification basis/quality of the identification basis, etc., which are transmitted from the server 1403, on the display screen of the image acquisition device 1405.

A regenerative medicine device with a photographing section, an iPS cell culture device, magnetic resonance imaging (MRI), an ultrasound image imaging device, or the like may be used as the image acquisition device 1405.

According to the third embodiment, for an object (e.g., tissue or cell) in an image transmitted from a facility at a different location, respective parameters of the identifier determined by the identifier creating device 1 are used to accurately classify whether the object is an object to be detected (such as abnormal tissue or cell), data, such as identification/determination results, an identification basis, and quality of the identification basis, are transmitted to a facility in a different location, and the data, such as the identification/determination results, the identification basis, and the quality of the identification basis, are displayed by the display section of an image acquisition device in that facility, making it possible to provide a remote diagnosis support system.

(4) Fourth Embodiment

Fig. 15 is a functional block diagram showing a configuration of an online contract service providing system 1500 according to a fourth embodiment of the Description. The online contract service providing system 1500 includes a server 1503 and an image acquisition device 1505. The server 1503 can be configured of, for example, one or more computers.

The image acquisition device 1505 is, for example, a virtual slide device or a personal computer equipped with a camera. The image acquisition device 1505 includes an imaging section 1501 to capture image data, a storage section 1504 to store an identifier transmitted from the server 1503, and an image diagnosis support device 2. The image diagnosis support device 2 reads the identifier transmitted from the server 1503, and for an object (e.g., tissue or cell) in an image newly captured by the imaging section 1501 of the image acquisition device 1505, classify whether the object is an object to be detected (such as abnormal tissue or cell) using the identifier obtained from the identifier creating device 1 of the first embodiment of the Description. Although not shown, the image acquisition device 1505 includes a communication device that transmits image data to the server 1503 and receives data transmitted from the server 1503.

The server 1503 includes a storage section 1502, which creates an identifier with the identifier creating device 1 of the first embodiment of the Description for image data transmitted from the image acquisition device 1505, and stores the identifier output from the identifier creating device 1. Furthermore, the server 1503 receives input designation of the teacher image from an operator, or creates the teacher image using the segmentation section 18. The server 1503 presents each output of the identifier creating device 1 (identification/determination results, identification basis, quality of the identification basis, etc.) to the operator. Although not shown, the server 1503 has a communication device that receives image data transmitted from the image acquisition device 1505 and transmits the identifier to the image acquisition device 1505.

The identifier creating device 1 performs machine learning on an object (e.g., tissue or cell) in image data captured by the imaging section 1501 so as to determine the object to be an object to be detected (e.g., a normal tissue or cell to be a normal tissue or cell, and an abnormal tissue or cell to be an abnormal tissue or cell), and creates an identifier to calculate feature amount of an object (e.g., tissue or cell) in an image in a facility at a different location. The storage section 1504 stores the identifier etc. transmitted from the server 1503.

The image diagnosis support device 2 in the image acquisition device 1505 reads the identifier etc. from the storage section 1504, uses the identifier to classify whether an object (e.g., tissue or cell) in the image, which is newly captured by the imaging section 1501 of the image acquisition device 1505, is an object to be detected (e.g., abnormal tissue or cell), and displays data, such as identification/evaluation results and identification basis/quality of the identification basis, on the display screen of the output unit 204 of the image diagnosis support device 2.

A regenerative medicine device with a photographing section, an iPS cell culture device, or an MRI or ultrasound image imaging device, etc. may be used as the image acquisition device 1505.

According to the fourth the embodiment, for an object (e.g., tissue or cell) in an image transmitted from a facility etc. at a different location, machine learning is performed to classify the object as an object to be detected (e.g., a normal tissue or cell to be a normal tissue or cell, and an abnormal tissue or cell as an abnormal tissue or cell) so that the identifier, etc. is created and transmitted to the facility etc. at the different location, and is read by an image acquisition device in that facility, and whether an object (e.g., tissue or cell) in a newly captured image is an object to be detected (e.g., abnormal tissue or cell) is classified, making it possible to provide an online contract service provision system.

The above-described embodiments can each be modified as follows. Although the feature extraction section 11 has determined multiple feature amounts using a filter through machine learning, it may use another feature amount such as Histogram of Oriented Gradient (HOG) to achieve similar effects. Although the identification section 12 has used Negative log likelihood as the loss function (cost), it may use square error, Hinge loss, etc., to achieve similar effects.

The invention can also be achieved by a software program code that implements the functions of the embodiments. In this case, a storage medium storing the program code is provided to a system or a device, and a computer (or CPU or MPU) of the system or the device reads the program code stored in the storage medium. In this case, the program code read from the storage medium implements the functions of the above embodiments by itself, and the program code itself and the storage medium storing the program code configure the invention. Examples of usable storage media for supplying such a program code include a flexible disk, CD-ROM, DVD-ROM, a hard disc, an optical disk, a magneto-optical disc, CD-R, a magnetic tape, a nonvolatile memory card, and ROM.

An operating system (OS) running on a computer may perform part or all of actual processing according to an instruction of the program code so that the functions of the above embodiments are implemented through such processing. Furthermore, after the program code read from the storage medium is written into a memory on a computer, CPU, etc. of the computer may perform part or all of the actual processing according to the instruction of the program code so that the functions of the embodiments may bee implemented through such processing.

Furthermore, the program code of the software, which implements the functions of the embodiments, may be distributed via a network, and stored in a storage tool such as a hard disc or memory of a system or device, or in a storage medium such as CD-RW or CD-R so that when the program code is used, the computer (or CPU or MPU) of the system or device reads and executes the program code stored in the storage tool or the storage medium.

Finally, the processes and techniques described herein are not inherently involved in any particular device, and can be implemented in any suitable combination of components. Furthermore, various types of general purpose devices can be used in accordance with the methods described herein. It may be beneficial to construct a dedicated device for performing the steps of the methods described herein. Various inventions can be constructed through appropriate combinations of multiple components disclosed in the embodiments. For example, some components may be removed from all the components shown in the embodiments. Furthermore, components in various embodiments may be combined as appropriate. Although the invention has been described in connection with the specific examples, the examples are in all respects for illustration rather than limitation. Those skilled in the art will appreciate that there are many combinations of hardware, software, and firmware that would be suitable for practicing the invention. For example, the described software may be implemented in a wide variety of programming or scripting languages, such as Assembler, C/C++, perl, Shell, PHP, Java (registered trademark), etc.

Furthermore, in the above embodiments, the control lines and the information lines are those considered necessary for illustrative purposes, and all control and information lines for the products are not necessarily shown. All components may be interconnected.

In addition, other implementations of the invention will be apparent to a person having ordinary skill in the art from consideration of the Description and embodiments of the invention disclosed herein. Various aspects and/or components of the described embodiments can be used alone or in any combination.

**Claims**

1. An identifier creating device, comprising:

   a processor executing a program to perform image processing on an objective image; and
   a memory to store the program and a result of the image processing,
   wherein the memory stores the objective image and an identifier, and
   the processor
   extracts, using the identifier, feature amount of the objective image from the objective image,
   identifies, using the identifier, the objective image from the feature amount and determines an identification value,
   determines an identification basis using the feature amount and the identification value,
   determines a portion to be improved using the identification basis and a teacher image, and
   updates the identifier based on the portion to be improved.

2. The identifier creating device according to claim 1,
   wherein when determining the portion to be improved, the processor analyzes a cause of misidentification of the identifier, and creates an analysis result and quality of the identification basis of the objective image.

3. The identifier creating device according to claim 1,
   wherein when updating the identifier, the processor uses cost based on an identification result of the identifier and cost based on the identification basis and the teacher image.

4. An image diagnosis support device, comprising:

   a processor executing a program to perform image processing on an objective image; and
   a memory to store the program and a result of the image processing,
   wherein the memory stores the objective image and an identifier, and

   the processor

   extracts, using the identifier, feature amount of the objective image from the objective image,
   identifies, using the identifier, the objective image from the feature amount and determines an identification value,
   determines an identification basis using the feature amount and the identification value,
   determines a portion of the identifier, the portion being to be improved, using the identification basis and a teacher image, and
   presents information based on the identification basis and the portion to be improved.

5. The image diagnosis support device according to claim 4,

   wherein the processor,
   when determining the portion to be improved, analyzes a cause of misidentification of the identifier, and creates an analysis result and quality of the identification basis of the objective image, and
   includes the quality of the identification basis in the information to be presented.

6. The image diagnosis support device according to claim 4,

   wherein the information to be presented includes the objective image and quality of the identification basis of the objective image, and
   when determining the portion to be improved, the processor compares performance values of the identifiers, determines performance of each identifier, and presents information of a determination result.

7. An identifier creating method where a system creates an identifier to classify a desired object in an objective image, the system comprising:

a processor executing a program to perform image processing on the objective image; and
a memory to store the program and a result of the image processing,
the memory storing the objective image and an identifier,
wherein, in the identifier creating method, the processor
extracts, using the identifier, feature amount of the objective image from the objective image,
identifies, using the identifier, the objective image from the feature amount and determines an identification value,
determines an identification basis using the feature amount and the identification value,
determines a portion of the identifier, the portion being to be improved, using the identification basis and a teacher image, and
updates the identifier based on the portion to be improved.

8. The identifier creating method according to claim 7,
wherein when determining the portion to be improved, the processor analyzes a cause of misidentification of the identifier, and creates an analysis result and quality of the identification basis of the objective image.

9. The identifier creating method according to claim 7,
wherein the processor updates the identifier using cost based on an identification result of the identifier and cost based on the identification basis and the teacher image.

10. An image diagnosis support method where a system classifies a desired object in an objective image, the system comprising:

a processor executing a program to perform image processing on the objective image; and
a memory to store the program and a result of the image processing,
the memory storing the objective image and an identifier,
wherein, the processor
extracts, using the identifier, feature amount of the objective image from the objective image,
identifies, using the identifier, the objective image from the feature amount and determines an identification value,
determines an identification basis using the feature amount and the identification value,
determines a portion of the identifier, the portion being to be improved, using the identification basis and a teacher image, and
presents information based on the identification basis and the portion to be improved.

11. The image diagnosis support method according to claim 10,

wherein when determining the portion to be improved, the processor analyzes a cause of misidentification of the identifier, and creates an analysis result and quality of the identification basis of the objective image, and
the information to be presented includes quality of the identification basis.

12. The image diagnosis support method according to claim 10,

wherein the information to be presented includes the objective image and quality of the identification basis of the objective image, and
when determining the portion to be improved, the processor compares performance values of the identifiers, determines performance of each identifier, and presents information of a determination result.

13. A remote diagnosis support system, comprising:

an image acquisition device including an imaging device to capture image data;
the identifier creating device according to claim 1; and
an image diagnosis support device,
wherein the image acquisition device transmits the image data to the identifier creating device,
the identifier creating device creates an identifier using the received image data, and
the image diagnosis support device acquires the identifier and an image of an object from the image acquisition device,

extracts, using the identifier, feature amount of the image of the object from the image of the object,

identifies, using the identifier, the object from the feature amount and determines an identification value and an identification result,

determines an identification basis using the feature amount and the identification value,

determines a portion of the identifier, the portion being to be improved, using the identification basis and a teacher image, and

transmits the identification result, and information based on the identification basis and the portion to be improved to the image acquisition device, and

the image acquisition device displays, on a display unit, the image of the object, the identification result, and the information based on the identification basis and the portion to be improved.

14. An online contract service offering system, comprising:

an image acquisition device including an imaging device to capture image data and an image diagnosis support device; and

the identifier creating device according to claim 1,

wherein the image acquisition device transmits the image data to the identifier creating device,

the identifier creating device creates an identifier using the received image data,

the identifier creating device transmits the created identifier to the image acquisition device, and

the image acquisition device stores the received identifier in a memory, and

the image diagnosis support device

acquires the identifier and an image of an object from the imaging device,

extracts, using the identifier, feature amount of the image of the object from the image of the object,

identifies, using the identifier, the object from the feature amount and determines an identification value and an identification result,

determines an identification basis using the feature amount and the identification value,

determines a portion of the identifier, the portion being to be improved, using the identification basis and a teacher image, and

displays, on a display unit, the image of the object, the identification result, and information based on the identification basis and the portion to be improved.

# FIG. 1

IMAGE DATA
(ex. MOVING IMAGE DATA)

1

CONTROL
SECTION ~19

CONNECTED TO
EACH ELEMENT

DOMAIN
SEGMENTATION
SECTION ~18

TEACHER IMAGE ~50

INPUT SECTION ~10

FEATURE
EXTRACTION
SECTION ~11

IDENTIFICATION
BASIS ANALYZATION
SECTION ~13

IDENTIFICATION
SECTION ~12

AUTOMATIC CAUSE
ANALYSIS SECTION ~14

OUTPUT
SECTION ~16

IDENTIFICATION
BASIS LEARNING
SECTION ~15

90

MEMORY

RECORDING
SECTION ~17

IDENTIFICATION RESULT

# FIG. 2

1

207 202

MEMORY

10

INPUT SECTION

14/24

AUTOMATIC CAUSE
ANALYSIS SECTION

201

CPU

11/21

FEATURE
EXTRACTION
SECTION

15

IDENTIFICATION
BASIS LEARNING
SECTION

204

OUTPUT UNIT

12/22

IDENTIFICATION
SECTION

16

OUTPUT SECTION

205

INPUT UNIT

13/23

IDENTIFICATION
BASIS ANALYZATION
SECTION

17

RECORDING
SECTION

18

DOMAIN
SEGMENTATION
SECTION

206

COMMUNICATION
DEVICE

203

AUXILIARY
STORAGE UNIT

# FIG. 3

INPUT IMAGE

FEA

FEATURE AMOUNT

A1

FEATURE EXTRACTOR

FAi

CNN
(FIRST LAYER)

→ ··· →

CNN
(NTH LAYER)

# FIG. 4

42 FILTER

41 OBJECTIVE IMAGE
(ex. PATHOLOGICAL
TISSUE IMAGE)

# FIG. 5

FEATURE AMOUNT

FAi

51

OBJECT TO BE DETECTED
(ABNORMAL TISSUE etc.)

NON-OBJECT TO BE DETECTED
(NORMAL TISSUE etc.)

LOGISTIC
REGRESSION
LAYER

# FIG. 6

INPUT
IMAGE
A1

FEA

FEATURE EXTRACTOR

CNN
(FIRST LAYER) → · · · → CNN
(NTH LAYER)

51

LOGISTIC
REGRESSION
LAYER

OBJECT TO
BE DETECTED

NON-OBJECT
TO BE
DETECTED

# FIG. 7

INPUT
IMAGE
71

73
ABNORMAL
TISSUE

72
NORMAL
TISSUE

→ IDENTIFICATION BASIS
ANALYZATION SECTION 13 →

IDENTIFICATION BASIS
75

77
78
79

BLACK: ABNORMAL TISSUE DOMAIN
WHITE, DOT: NORMAL TISSUE DOMAIN

# FIG. 8

TEACHER IMAGE
(CORRECT PATTERN)
81

IDENTIFICATION
BASIS ANALYZATION
SECTION 13

IDENTIFICATION
BASIS
75

→ AUTOMATIC
CAUSE ANALYSIS
SECTION 14 →

IDENTIFICATION
BASIS
75

MISIDENTIFICATION
CAUSE PORTION
82
83

BLACK: ABNORMAL
TISSUE DOMAIN

WHITE, DOT: NORMAL
TISSUE DOMAIN

BLACK: ABNORMAL
TISSUE DOMAIN

WHITE, DOT: NORMAL
TISSUE DOMAIN

# FIG. 9A

91   92   93   94

·OBJECT-LIKELIHOOD
(LESION-LIKELIHOOD)
DETERMINATION

■ BREAST

NON-
TUMOR   TUMOR

OBJECT-
LIKELIHOOD   DISPLAY   DISPLAY

○   ●   | 0.89 |   IMAGE   IDENTIFICATION
REASON

OBJECT-LIKELIHOOD DETERMINATION RESULT : TUMOR          TENFOLD IMAGE

# FIG. 9B

INPUT IMAGE                    IDENTIFICATION REASON

IMAGE

⟷

IDENTIFICATION
SCORE
: 0.89

QUALITY OF          IDENTIFICATION
IDENTIFICATION BASIS          BASIS

·NON-TUMOR : 0.96

·TUMOR : 0.93

97                                          98

# FIG. 10A

INPUT IMAGE                    101

TUMOR

103
LESION-LIKELIHOOD
DETERMINATION
RESULT

102
REGION SUSPICIOUS FOR
ABNORMAL TISSUE OR CELL

# FIG. 10B

106

· IDENTIFIER PERFORMANCE
  DETERMINATION              PERFORMANCE
                                VALUE
  ▨ IDENTIFIER A              1.97

                             PERFORMANCE
                                VALUE
  ▨ IDENTIFIER B              1.65

IDENTIFIER PERFORMANCE
DETERMINATION RESULT : IDENTIFIER A

# FIG. 11

START

OUTPUT INPUT IMAGE FOR LEARNING TO FEATURE EXTRACTION SECTION — S1101

OBTAIN FEATURE AMOUNT FAi OF OBJECT USING FILTER ACCORDING TO FORMULA 1 AND CREATE FEATURE EXTRACTOR A, AND CALCULATE FILTER COEFFICIENT wj AND OFFSET VALUE bi — S1102

CALCULATE IDENTIFICATION VALUE OF OBJECT, WEIGHT w, AND OFFSET VALUE b USING FEATURE AMOUNT FAi AND FILTER ACCORDING TO FORMULA 2 — S1103

CALCULATE IDENTIFICATION BASIS ACCORDING TO FORMULAS 3 AND 4 — S1104

CALCULATE MISIDENTIFICATION CAUSE SCORES $e_{back}$, $e_{fore}$ USING IDENTIFICATION BASES AND TEACHER IMAGE ACCORDING TO FORMULAS 5 AND 6, AND CALCULATE QUALITIES $coin_{back}$, $coin_{fore}$ OF IDENTIFICATION BASES ACCORDING TO FORMULAS 7 AND 8 — S1105

CALCULATE TOTAL COST cost OF EQUATION 12 ACCORDING TO FORMULAS 9, 10, AND 11, UPDATE FILTER COEFFICIENT wj, WEIGHT w, AND OFFSETS bi AND b, AND CREATE IDENTIFIER — S1106

STORE PARAMETERS (FILTER COEFFICIENT wj, WEIGHT w, OFFSET VALUES bi, b) OF FORMULAS 1 AND 2, IDENTIFICATION BASIS, AND QUALITY OF IDENTIFICATION BASIS IN MEMORY — S1107

END

# FIG. 12

IMAGE DATA
(ex. MOVING IMAGE DATA)

2

CONTROL SECTION ~19

CONNECTED TO
EACH ELEMENT

DOMAIN SEGMENTATION SECTION ~18

INPUT SECTION ~10

TEACHER IMAGE ~50

FEATURE EXTRACTION SECTION ~21

IDENTIFICATION BASIS ANALYZATION SECTION ~23

IDENTIFICATION SECTION ~22

AUTOMATIC CAUSE ANALYSIS SECTION ~24

OUTPUT SECTION ~16

90

MEMORY

RECORDING SECTION ~17

IDENTIFICATION RESULT

25

# FIG. 13

START

OUTPUT EVALUATION IMAGE TO FEATURE
EXTRACTION SECTION ~S1301

READ FILTER COEFFICIENT wj OF FEATURE
EXTRACTOR A AND OFFSET VALUE bi FROM
MEMORY, AND CALCULATE FEATURE AMOUNT FAi
OF OBJECT USING FILTER ACCORDING TO
FORMULA 1 ~S1302

READ WEIGHT w AND OFFSET VALUE b OF
IDENTIFIER FROM MEMORY, AND CALCULATE
IDENTIFICATION RESULT AND IDENTIFICATION
VALUE OF OBJECT FROM FEATURE AMOUNT FAi
ACCORDING TO FORMULA 2 ~S1303

CALCULATE IDENTIFICATION BASIS ACCORDING TO
FORMULAS 3 AND 4 ~S1304

CALCULATE MISIDENTIFICATION CAUSE SCORES
$e_{back}$, $e_{fore}$ USING IDENTIFICATION BASIS AND
TEACHER IMAGE CREATED BY DOMAIN
SEGMENTATION SECTION ACCORDING TO
FORMULAS 5 AND 6, AND CALCULATE QUALITIES
$coin_{back}$, $coin_{fore}$ OF IDENTIFICATION BASES
ACCORDING TO FORMULAS 7 AND 8 ~S1305

STORE IDENTIFICATION RESULTS, IDENTIFICATION
VALUES, IDENTIFICATION BASES, AND QUALITIES
OF IDENTIFICATION BASES IN MEMORY ~S1306

END

# FIG. 14

1405

1403

IMAGE ACQUISITION DEVICE

1401

IMAGING SECTION

TRANSMISSION (INPUT/ TEACHER IMAGE)

SERVER

1

IDENTIFIER CREATING DEVICE

2

IMAGE DIAGNOSIS SUPPORT DEVICE

IDENTIFICATION/ DETERMINATION RESULT/ IDENTIFIER

1402

1404

DISPLAY SECTION

TRANSMISSION (IDENTIFICATION/ DETERMINATION RESULT)

STORAGE SECTION

1400

REMOTE DIAGNOSIS SUPPORT SYSTEM

# FIG. 15

OPERATOR

INPUT EACH OPERATION OUTPUT

1503

1505

IMAGE ACQUISITION DEVICE

1501

IMAGING SECTION

TRANSMISSION (INPUT IMAGE)

SERVER

1

IDENTIFIER CREATING DEVICE

IDENTIFIER

1502

1504

STORAGE SECTION

TRANSMISSION (IDENTIFIER)

STORAGE SECTION

2

IMAGE DIAGNOSIS SUPPORT DEVICE

1500

ONLINE CONTRACT SERVICE OFFERING SYSTEM

<div align="center">**INTERNATIONAL SEARCH REPORT**</div>

| | International application No. |
|---|---|
| | **PCT/JP2023/021065** |

### A. CLASSIFICATION OF SUBJECT MATTER

*G06T 7/00*(2017.01)i; *G06V 10/778*(2022.01)i
FI: G06T7/00 350B; G06T7/00 612; G06V10/778

According to International Patent Classification (IPC) or to both national classification and IPC

### B. FIELDS SEARCHED

Minimum documentation searched (classification system followed by classification symbols)

G06T7/00; G06V10/778

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2022/059315 A1 (NATIONAL CANCER CENTER) 24 March 2022 (2022-03-24) paragraphs [0057]-[0060], [0068]-[0076] | 1-5, 7-11 |
| Y | | 6, 12-14 |
| Y | JP 2021-189960 A (HITACHI HIGH-TECH CORPORATION) 13 December 2021 (2021-12-13) paragraphs [0032], [0045], [0104]-[0107] | 6, 12 |
| Y | JP 2021-152692 A (UNIV SAITAMA) 30 September 2021 (2021-09-30) paragraph [0056] | 13-14 |
| Y | JP 2020-194446 A (ARK JOHO SYSTEMS KK) 03 December 2020 (2020-12-03) paragraph [0029] | 13-14 |
| Y | JP 2022-80063 A (SUMITOMO RUBBER IND) 27 May 2022 (2022-05-27) paragraph [0045] | 13-14 |
| Y | JP 2021-140270 A (OMRON TATEISI ELECTRONICS CO) 16 September 2021 (2021-09-16) paragraphs [0074], [0093]-[0094] | 13-14 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 August 2023** | **22 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/021065**

| Patent document cited in search report | | | Publication date (day/month/year) | Patent family member(s) | | | Publication date (day/month/year) |
|---|---|---|---|---|---|---|---|
| WO | 2022/059315 | A1 | 24 March 2022 | (Family: none) | | | |
| JP | 2021-189960 | A | 13 December 2021 | WO | 2021/246013 | A1 | |
| | | | | CN | 115699084 | A | |
| JP | 2021-152692 | A | 30 September 2021 | (Family: none) | | | |
| JP | 2020-194446 | A | 03 December 2020 | (Family: none) | | | |
| JP | 2022-80063 | A | 27 May 2022 | (Family: none) | | | |
| JP | 2021-140270 | A | 16 September 2021 | (Family: none) | | | |

Form PCT/ISA/210 (patent family annex) (January 2015)

**REFERENCES CITED IN THE DESCRIPTION**

**Patent documents cited in the description**

- JP 2022099357 A
- JP 2016143351 A